# EUROPÄISCHE PATENTANMELDUNG

(11) **EP 2 974 731 A1**
(43) Veröffentlichungstag der Anmeldung: **20.01.2016**
(21) Anmeldenummer: 14177880.3
(22) Anmeldetag: 21.07.2014
(51) Int. Cl.: A61K 35/12, A61K 8/67, A61K 31/593, A61K 33/00, A61K 36/06, A61P 1/00, A61P 5/24, A61P 9/00, A61P 19/00, A61P 25/00

(54) **Verabreichungsform enthaltend Pilzkomponenten**

(30) Priorität: 18.07.2014 EP 14177699
(71) Anmelder: Hennig Arzneimittel GmbH&Co. Kg, 65439 Flörsheim am Main (DE)
(72) Erfinder: Schleenhain, Holger, 65439 Flörsheim am Main (DE); Dr. Schleenhain, Kai, 65439 Flörsheim am Main (DE)
(74) Vertreter: Fuchs Patentanwälte Partnerschaft mbB

(57) **Zusammenfassung**

Die vorliegende Erfindung betrifft eine Verabreichungsform umfassend zwei Einheiten A und B, deren Zusammensetzungen so an den Tag-Nacht-Rhythmus angepasst sind, dass die Verabreichungsform zu einer spürbaren Steigerung des Wohlbefindens beitragen kann. Die Verabreichungsform kann flexibel formuliert werden, so dass verschiedene Anwendungsmöglichkeiten bestehen. Diese sind beispielsweise: Steigerung der Gedächtnisleistung, Stärkung des Herz Kreislaufsystems, Unterstützung der Verdauungsfunktion, Linderung von Beschwerden in der Prämenopause und Menopause, Förderung eines gesunden Gelenk- und Knochenapparates.

## Beschreibung

Um zu überleben, sind Menschen und andere Lebewesen auf Nahrung angewiesen. Insbesondere werden unter Nahrung energiereiche organische Verbindungen wie beispielsweise Fette, Kohlenhydrate und Proteine verstanden, die aufgenommen werden, um den Energiebedarf zu decken. Darüber hinaus gehören aber auch Wasser, Mineralstoffe und Vitamine zur Nahrung. Vitamine sind Verbindungen, die für ein Lebewesen essentiell sind, von diesem jedoch nicht selbst synthetisiert werden können. Zur Nahrung gehören auch weitere Stoffe und Verbindungen, deren Wirkungen auf den Organismus bisher wenig bekannt sind. Insbesondere pflanzliche Nahrung enthält eine Reihe solcher Verbindungen mit wenig erforschter Wirkung.

Die Nahrung ist nicht nur für das Überleben, sondern auch für das Wohlbefinden wichtig. Insbesondere schlechte und einseitige Ernährung kann mit einer Reihe von Beeinträchtigungen einhergehen. Daher wurden seit längerer Zeit Versuche unternommen, die Nahrung durch Nahrungsergänzungsmittel gezielt zu ergänzen, um durch eine ausreichende Zufuhr von ansonsten zu wenig oder gar nicht aufgenommenen Stoffen derartigen Beeinträchtigungen vorzubeugen oder diese zu beheben. Nahrungsergänzungsmittel enthalten in der Regel in erster Linie eine Kombination verschiedener Vitamine. Obwohl unzählige Nahrungsergänzungsmittel erhältlich sind, wurden wirklich befriedigende Ergebnisse jedoch bisher nicht erzielt.

In den letzten zehn bis fünfzehn Jahren ist die sogenannte Chronobiologie vermehrt in den Fokus der Ernährungswissenschaften gerückt. Es war seit längerer Zeit bekannt, dass eine Vielzahl physiologischer Prozesse einem circadianen Rhythmus unterliegt. Dennoch wurde die Bedeutung dieser tageszeitabhängigen Schwankungen für die Ernährung lange nicht in ihrer ganzen Tragweite erkannt. Es wurde zwar vermutet, dass eine späte Nahrungsaufnahme kurz vor dem Schlaf mit einem erhöhten Risiko für Fettleibigkeit verbunden ist. Diese Vermutung wurde jedoch nicht überzeugend empirisch belegt. Die Idee einer gezielten Anpassung der Gabe von Nahrung und Nahrungsergänzungsmitteln an den circadianen Rhythmus wurde nur sehr vereinzelt verfolgt.

US 5,895,652 A offenbart eine Vielzahl von Nahrungsergänzungsmitteln. Es wird eine dreimal tägliche Einnahme vorgeschlagen, wobei sich die morgens, mittags und abends eingenommenen Nahrungsergänzungsmittel hinsichtlich ihrer Zusammensetzung unterscheiden können. Als Inhaltsstoffe werden Vitamine, Mineralstoffe und Pflanzenextrakte genannt. Die offenbarten Nahrungsergänzungsmittel sollen geeignet sein, den Alterungsprozess zu verlangsamen.

Eine dreimal tägliche Einnahme wird im Allgemeinen als kompliziert empfunden und daher oft nicht regelmäßig genug, wenn überhaupt vorgenommen. Insbesondere eine Einnahme zur Mittagszeit wird häufig vernachlässigt, da sich diese regelmäßig mit der Arbeitszeit überschneidet.

Trotz einiger Versuche der Berücksichtigung der Erkenntnisse der Chronobiologie bei der Entwicklung von Nahrungsergänzungsmitteln ist daher bis heute kein Nahrungsergänzungsmittel bekannt, dessen Zusammensetzung und Dosierungsregime in zufriedenstellender Art und Weise an den ernährungsphysiologischen Tag-Nacht-Rhythmus angepasst sind und das so zu einer spürbaren Steigerung des Wohlbefindens beiträgt.

Es ist daher eine Aufgabe der vorliegenden Erfindung ein Nahrungsergänzungsmittel bereitzustellen, das die Nachteile des Stands der Technik überwindet. Die Aufgabe wird durch die Gegenstände der Ansprüche gelöst.

Die Aufgabe wird insbesondere gelöst durch eine Verabreichungsform umfassend zwei Einheiten A und B, die unabhängig voneinander verabreicht werden können, wobei die Einheiten A und B jeweils eine Pilzkomponente enthalten und ferner mindestens zwei weitere Substanzen ausgewählt aus Naturstoffextrakten, Vitaminen und Mineralstoffen, wobei sich die Einheiten A und B bezüglich ihrer Zusammensetzung voneinander unterscheiden.

Gemäß besonderen Ausführungsformen der Erfindung enthalten die Einheiten A und B der Verabreichungsform abschließend mindestens eine Pilzkomponente und mindestens zwei weitere Komponenten, ausgewählt aus einem Naturstoffextrakt, einem Vitamin und einem Mineralstoff, zudem übliche pharmazeutische Zusatzstoffe, wie insbesondere mikrokristalline Cellulose, welchen Zusatzstoffen keine physiologische Wirkung zugeordnet wird, sowie optional Co-Enzym Q-10.

Gemäß besonderen Ausführungsformen der Erfindung enthalten die Einheiten A und B der Verabreichungsform abschließend mindestens eine Pilzkomponente, mindestens einen Naturstoffextrakt, mindestens ein Vitamin und mindestens einen Mineralstoff, sowie übliche pharmazeutische Zusatzstoffe, wie insbesondere mikrokristalline Cellulose, welchen Zusatzstoffen keine physiologische Wirkung zugeordnet wird, und optional Co-Enzym Q10.

Die beiden Einheiten der Verabreichungsform können dergestalt flexibel formuliert werden, dass die Verabreichungsform auf verschiedene Anwendungsmöglichkeiten abgestimmt ist. Anwendungsmöglichkeiten sind beispielsweise: Steigerung der Gedächtnisleistung, Stärkung des Herz Kreislaufsystems, Unterstützung der Verdauungsfunktion, Linderung von Beschwerden in der Prämenopause und Menopause, Förderung eines gesunden Gelenk- und Knochenapparates. Für alle Anwendungsmöglichkeiten sind die Einheiten A und B der Verabreichungsform aufeinander abgestimmt. Nämlich derartig, dass die Einnahme zwei Mal täglich, insbesondere morgens und abends, es ermöglicht, dass die gewünschten Wirkungen an den circadianen Rhythmus des Menschen angepasst sind. Üblicherweise wird die Einheit A der erfindungsgemäßen Verabreichungsform morgens und die Einheit B der erfindungsgemäßen Verabreichungsform abends eingenommen.

### Die Darreichungsform

Erfindungsgemäß werden die Einheiten der Verabreichungsformen bevorzugt als Kapsel ausgebildet. Es ist jedoch jede andere feste abgeteilte Darreichungsform denkbar. Dazu gehören insbesondere Tabletten, Dragees und andere dem Fachmann bekannte Darreichungsformen.

Die erfindungsgemäß bevorzugten Kapseln umfassen eine äußere Hülle, die bevorzugt aus zwei Teilen besteht. Die beiden Teile fügen sich zu einer Kapsel zusammen. Besonders bevorzugt ist, dass eine Hälfte der Kapsel durchsichtig und die andere Hälfte farbig ausgestaltet ist. Bevorzugt sind die Einheiten A und B derartige Kapseln. Dabei ist es möglich, dass die farbige Hälfte der Einheiten A und B unterschiedlich ausgestaltet ist. Gemäß einer bevorzugten Ausführungsform sind die unterschiedlichen Einheiten A und B, die auch farblich unterschiedlich gestaltet sind, in einem Blister verblistert. Erfindungsgemäß wird unter Einnahme zu verschieden Zeitpunkten verstanden, dass eine Einheit, beispielsweise die Einheit A, der Verabreichungsform und eine weitere Einheit, beispielsweise die Einheit B, der Verabreichungsform an einem Tag mit einem zeitlichen Abstand von mindestens 7 Stunden, auch 8 Stunden oder 9 Stunden eingenommen werden. Der zeitliche Abstand wird üblicherweise maximal 14 Stunden, aber auch 13 Stunden, 12 Stunden, 11 Stunden und auch 10 Stunden betragen. In der Regel wird die Einheit A der Verabreichungsform mit dem Frühstück und die Einheit B der Verabreichungsform mit dem Abendessen eingenommen. Diese Mahlzeiten finden bekannter Weise zu verschiedenen Zeitpunkten am Tag, je nach Lebensrhythmus eines Menschen, statt. Vorzugsweise werden die jeweiligen Einheiten der Verabreichungsformen mit der Mahlzeit eingenommen. Eine Einheit, die erfindungsgemäß eine Kapsel sein kann, wird zu der entsprechenden Mahlzeit und der entsprechenden Tageszeit mit einer geeigneten Menge Wasser geschluckt. Eine geeignete Menge ist beispielsweise ein halbes Glas Wasser oder etwa 100 ml Wasser. Es gibt Verwendungsmöglichkeiten für die erfindungsgemäßen Einheiten A und B der Verabreichungsform, für die es sinnvoll ist, dass zu einem Zeitpunkt auch mehr als eine Einheit der Verabreichungsform eingenommen wird. Dies können beispielsweise zwei Einheiten, insbesondere zwei Kapseln sein. Es ist denkbar, dass beispielsweise morgens zwei Einheiten A und abends zwei Einheiten B eingenommen werden.

### Die Pilzkomponente

Die Pilzkomponente gemäß der Erfindung ist ein Pilzextrakt oder Pilzpulver.

Vorzugsweise handelt es sich bei den Pilzkomponenten um Pulver oder Extrakte chinesischer Pilze, die auch als chinesische Vitalpilze bezeichnet werden. Diese sind bevorzugt ausgewählt aus Cordyceps sinensis, Shiitake, Maitake, Auricularia polytricha, Hericium erinaceus, Coprinus comatus, Coriolus versicolor, Pleurotus ostreatus, Polyporus umbellatus, Reishi-Ganoderma lucidum, Agaricus blazei Murrill, und Mischungen aus zwei oder mehreren. Es sind auch andere geeignete Pilze erfindungsgemäß einsetzbar. Eine weiter bevorzugte Gruppe von Pilzen ist Cordyceps sinensis, Shütake, Maitake, Auricularia polytricha, Hericium erinaceus und Mischungen aus zwei oder mehreren. Gemäß besonderen Ausführungsformen enthält mindestens eine Einheit und möglicherweise beide Einheiten der Verabreichungsform eine einzige Pilzkomponente. Diese kann sein Cordyceps sinensis. Eine alternative Variante ist eine Verabreichungsform, bei welcher mindestens eine Einheit nur Shiitake als Pilzkomponente enthält. Denkbar ist auch eine Verabreichungsform, bei welcher mindestens eine Einheit nur Maitake als Pilzkomponente enthält. Ebenfalls erfindungsgemäß ist eine Verabreichungsform, bei welcher mindestens eine Einheit nur Auricularia polytricha als Pilzkomponente enthält. Eine weitere erfindungsgemäße Verabreichungsform enthält nur Hericium erinaceus als Pilzkomponente in mindestens einer Einheit.

Die beiden Einheiten A und B unterscheiden sich hinsichtlich Ihrer Zusammensetzungen. Es ist möglich, jedoch nicht zwingend, dass sich die Zusammensetzungen der Einheiten A und B dadurch unterscheiden, dass die Pilzkomponenten verschieden sind. Es kann beispielsweise eine Einheit A mindestens eine Pilzkomponente und eine Einheit B mindestens eine Pilzkomponente enthalten, wobei sich die Pilzkomponenten voneinander unterscheiden. Es ist denkbar, dass jede Einheit A und B genau eine Pilzkomponente enthält, wobei sich die Pilzkomponenten voneinander unterscheiden. Eine unterschiedliche Zusammensetzung kann aber auch dergestalt sein, dass die Einheiten A und B mindestens eine Pilzkomponente enthalten, wobei sich lediglich die Menge der mindestens einen Pilzkomponente unterscheidet. Wie weiter unten ausgeführt, kann die unterschiedliche Zusammensetzung der Einheiten A und B auch durch den unterschiedlichen Einsatz anderer Substanzen als der Pilzkomponente bedingt sein.

Cordyceps sinensis ist ein chinesischer Pilz und bekannt unter den weiteren Bezeichnungen chinesischer Raupenpilz, tibetischer Raupenkeulenpilz, Ophiocordyceps sinensis. In der TCM (traditionellen chinesischen Medizin) wird der Raupenpilz seit Jahrhunderten als Tonikum eingesetzt. In der Praxis erprobte Anwendungsgebiete sind beispielsweise die Steigerung von Leistungsfähigkeit und Ausdauer, die Stärkung des Immunsystems, die Behebung sexueller Funktionsstörungen und Steigerung der Libido, Stimmungsaufhellung, Regulierung der Herz- und Lungenfunktionen und Muskelregeneration. Da der Raupenpilz überwiegend anregend wirkt, soll die Einnahme morgens erfolgen, damit der Schlaf nicht beeinträchtigt wird.

Pilzpulver oder Pilzextrakt von Cordyceps sinensis werden pro Einheit in Mengen von 100 bis 300 mg eingesetzt. Denkbar sind auch Mengen von 150 bis 250 mg. Eine gängige Menge beträgt 200 mg Pilzkomponente des Cordyceps sinensis.

Shiitake ist ein chinesischer Pilz und bekannt unter den weiteren Bezeichnungen Pasaniapilz, Shaingugu, Hua Gu, Qua Gu, forest mushroom, oak mushroom. In der Praxis erprobte Anwendungsgebiete sind unter anderem Linderung der Beschwerden bei Osteoporose und rheumatischen Erkrankungen, beispielsweise durch Anregung der Kollagenproduktion. Zudem enthält der Pilz Vitamin D, was den Calciumstoffwechsel fördert. Calcium wird für die Erhaltung normaler Knochen benötigt. Aufgrund der aufbauenden und regenerierenden Wirkung wird Shiitake abends eingesetzt.

Pilzpulver oder Pilzextrakt von Shiitake werden pro Einheit in Mengen von 150 bis 350 mg eingesetzt. Denkbar sind auch Mengen von 200 bis 300 mg. Eine gängig Menge beträgt 280 mg Pilzkomponente des Shiitake.

Maitake ist ein chinesischer Pilz und bekannt unter den weiteren Bezeichnungen Klapperschwamm, Tanzender Pilz, Laubporling, Hen-of-the-Woods, Kumotake.

Maitake enthält unter anderem Ergosterin, eine Vorstufe des Vitamins D, Riboflavin, Niacin, Thiamin, Biotin und Folsäure, Mineralstoffe und Spurenelemente.

Wirkungen sind beispielsweise die Stärkung des Immunsystems und die Unterstützung des Skelettsystems bei Osteoporose.

Pilzpulver oder Pilzextrakt von Maitake werden pro Einheit in Mengen von 100 mg bis 300 mg eingesetzt. Denkbar sind auch Mengen von 120 mg bis 250 mg. Gängige Mengen sind auch 140 bis 230 mg.

Auricularia polytricha ist ein chinesischer Pilz und bekannt unter den weiteren Bezeichnungen Chinesische Morchel, Holunderpilz, Mu Er (Mu Err, Mu Erh), Judasohr.

In der Praxis erprobte Anwendungsgebiete sind unter anderem die Förderung der Durchblutung, zum Beispiel bei Arteriosklerose; Regulation des Blutdrucks; Reduktion der Blutgerinnung, dadurch vorbeugende Wirkung gegen Herzinfarkt, Schlaganfall und Thrombose. Im Gegensatz zu chemisch-pharmazeutischen Blutverdünnern kann der Auricularia die Fließeigenschaften des Blutes verbessern, ohne die Gefäßwände anzugreifen. Zur Verbesserung der Fließeigenschaften während der Immobilisation in der Schlafphase wird Auricularia vorzugsweise abends eingenommen.

Pilzpulver oder Pilzextrakt von Auricularia werden pro Einheit in Mengen von 100 bis 300 mg eingesetzt. Denkbar sind auch Mengen von 150 bis 250 mg. Eine gängige Menge an Auricularia beträgt 210 mg Pilzkomponente.

Hericium erinaceus ist ein chinesischer Pilz und bekannt unter den weiteren Bezeichnungen Igelstachelbart, Knolliger Stachelbart, Affenkopfpilz, Löwenmähne, Pom-Pom, Yamabushitake, Houtou. In der Praxis erprobte Anwendungsgebiete sind beispielsweise Regulation von Magen- und Darmproblemen, Begleitende Behandlung bei Nervenerkrankungen, Beruhigung bei Ängsten, innerer Unruhe und Schlafstörungen.

Es unterscheidet sich das Anwendungsspektrum von Pulver und Extrakt. Und das sowohl bezüglich der gewünschten Wirkung als auch bezüglich des Zeitpunktes der Einnahme.

Das Pulver wird vorzugsweise bei Beschwerden des Verdauungstraktes eingesetzt. Es beruhigt die Schleimhäute, was sich günstig bei einer Gastritis auswirkt. Daher wird das Pulver vorzugsweise tagsüber eingesetzt.

Der Extrakt stimuliert die Regeneration von Nervengewebe und wirkt beispielsweise stimmungsaufhellend und zur Unterstützung bei innerer Unruhe und Schlafstörungen. Daher wird der Extrakt vorzugsweise abends eingesetzt.

Hericium erinaceus wird pro Einheit in Mengen von 50 bis 400 mg eingesetzt. Denkbar sind auch Mengen von 100 bis 200 mg. Eine gängige Menge an Hericium erinaceus beträgt 270 oder auch 300 mg.

### Naturstoffextrakte

Die Einheiten der Verabreichungsform können Naturstoffextrakte enthalten.

Naturstoffextrakte gemäß der Erfindung sind beispielsweise aber nicht abschließend Pflanzenextrakte oder Extrakte aus tierischen Produkten, wie beispielsweise Bestandteile von Eiern, insbesondere Hühnereiern. Gemäß einer besonders bevorzugten Ausführungsform wird ein Naturstoffextrakt aus der Eihautmembran eines unbebrüteten Hühnereis gewonnen.

Die Naturstoffextrakte können Pflanzenextrakte sein. Bevorzugte Pflanzenextrakte sind ausgewählt aus der Gruppe bestehend aus Hagebuttenextrakt, Traubenkernextrakt, Weißdornfruchtextrakt, Melissenextrakt, Yamswurzelextrakt, Sojaextrakt, Rotkleeextrakt, Ginsengextrakt, Rosenwurzextrakt, Artischockenextrakt (gewonnen aus den Schuppenblättern und nicht den Laubblättern), Fenchelextrakt (gewonnen aus den Samen), Ginkgoextrakt, Bambussprossenextrakt, Haferextrakt, Brennesselextrakt, Brokkoliextrakt und Mischungen aus zwei oder mehreren daraus. Eine bevorzugte Gruppe von Pflanzenextrakten ist ausgewählt aus der Gruppe bestehend aus Hagebuttenextrakt, Traubenkernextrakt, Weißdornfruchtextrakt, Melissenextrakt, Yamswurzelextrakt, Sojaextrakt, Rotkleeextrakt, Ginsengextrakt, Rosenwurzextrakt, Artischockenextrakt (Schuppenblätter nicht Laubblätter), Fenchelextrakt (Samen), Ginkgoextrakt und Mischungen aus zwei oder mehreren daraus.

Artischockenextrakt enthält unter anderem folgend Inhaltsstoffe: Bitterstoffe (Sesquiterpenlactone), Chinasäure-Derivate, Flavonoide. Zur Wirkung gehört beispielsweise die Förderung von Gallenbildung und Gallenfluß (Förderung der Fettverdauung und Entgiftung, Verstärkung der Cholesterin-Ausscheidung (Gallensäuren), Hemmung der Cholesterin-Synthese (HMG-CoA-Reduktase Hemmung), antioxidative Wirkung (Hemmung der LDL-C Oxidation).

Tagsüber gewünscht wird die Förderung der Fettverdauung, nämlich Anregung des Gallenflusses.

Artischockenextrakt wird pro Einheit in Mengen von 150 bis 350 mg eingesetzt. Denkbar sind auch Mengen von 200 bis 300 mg. Eine gängige Menge an Artischockenextrakt beträgt 270 mg.

Fenchelextrakt enthält unter anderem folgende Inhaltsstoffe: Ätherische Öle, z.B. Anethol, Fettlösliche Phenylpropane und Terpene. Zur Wirkung, die insbesondere abends gewünscht ist, gehört unter anderem der spasmolytische Effekt auf die Darmmuskulatur sowie die Verminderung von Gärungsgasen.

Fenchelextrakt wird pro Einheit in Mengen von 100 bis 300 mg eingesetzt. Denkbar sind auch Mengen von 150 bis 250 mg. Eine gängige Menge an Fenchelextrakt beträgt 200 mg.

Ginsengextrakt enthält unter anderem folgende Inhaltsstoffe: Saponine, insbesondere Triterpensaponine.

Die tagsüber erwünschten Wirkungen sind insbesondere adaptogen stärkende Effekte, positive Wirkung auf die Stimmungslage und kognitive Funktionen, positive Wirkung auf das Lernvermögen. Ginsengextrakt wirkt ferner immunmodulierend und vermindert damit stressbedingte Infektionskrankheiten. Das Immunsystem ist Angriffen durch Krankheitserreger vorwiegend tagsüber ausgesetzt.

Ginsengextrakt wird pro Einheit in Mengen von 30 bis 250 mg eingesetzt. Denkbar sind auch Mengen von 80 bis 150 mg. Eine gängige Menge an Ginsengextrakt beträgt 100 mg.

Rosenwurzextrakt enthält unter anderem folgende Inhaltsstoffe: Phenylpropanderivate, Phenylethylderivate, Flavonoide, Monoterpene, Triterpene, Phenolsäuren.

In der russischen Medizin gilt die Pflanze seit langer Zeit als Adaptogen, das heißt, die Pflanze soll helfen, sich an veränderte Situationen, Stress und Belastungen anzupassen. Dabei steigert sie die geistige und körperliche Leistungsfähigkeit, die Widerstandskraft und bei älteren Menschen unterstützt sie durch ihre Wirkung die geistige Agilität und steigert die Leistungsfähigkeit. Rosenwurzextrakt dient ferner dem Stressabbau. Daher wird der Extrakt vorzugsweise morgens eingenommen.

Rosenwurzextrakt wird pro Einheit in Mengen von 50 bis 250 mg eingesetzt. Denkbar sind auch Mengen von 100 bis 200 mg. Eine gängige Menge an Rosenwurzextrakt beträgt 150 mg.

Ginkgoextrakt enthält unter anderem folgende Inhaltsstoffe: Flavonoide (gefäßverstärkend), Terpenoide (Ginkgolide, Bilobaid).

Die Wirkungen sind beispielsweise durchblutungsfördernd und die Thrombozytenaggregation hemmend, ferner auch neuroprotektiv. Der neuroprotektive Effekt ist insbesondere nachts erwünscht.

Ginkgoextrakt wird pro Einheit in Mengen von 30 bis 200 mg eingesetzt. Denkbar sind auch Mengen von 50 bis 150 mg. Eine gängige Menge an Ginkgoextrakt beträgt 80 mg.

Traubenkernextrakt enthält unter anderem folgende Inhaltsstoffe: Oligomere Proanthocyanidine (OPCs), die zu den Polyphenolen gehören.

Deren hohes antioxidatives Potential sowie die entzündungshemmende Wirkung sind vorwiegend tagsüber erwünscht.

Traubenkernextrakt wird pro Einheit in Mengen von 100 bis 300 mg eingesetzt. Denkbar sind auch Mengen von 150 bis 250 mg. Eine gängige Menge an Traubenkernextrakt beträgt 200 mg.

Weißdornextrakt enthält unter anderem folgende Inhaltsstoffe: Oligomere Procyanidine und Flavonoide. Die Wirkungen sind beispielsweise die Steigerung der Herz-Kontraktionskraft (positiv inotrop), Erweiterung der (Herzkranz-)Gefäße, verbesserte Myocard-Durchblutung und Koronargefäße, Toleranzsteigerung des Myocards gegen O₂-Mangel. Eine gute Durchblutung ist insbesondere während der Nachtzeit in der Immobilisation erwünscht.

Weißdornextrakt wird pro Einheit in Mengen von 50 bis 200 mg eingesetzt. Denkbar sind auch Mengen von 800 bis 150 mg. Eine gängige Menge an Weißdornextrakt beträgt 100 mg.

Melissenextrakt enthält unter anderem folgende Inhaltsstoffe: Ätherische Öle, Gerbstoffe, Bitterstoffe, Flavonoide und Rosmarinsäure.

Traditionell werden Melissenblätter zur Verbesserung des Befindens bei nervlicher Belastung angewendet. Melissenpräparate wirken beruhigend ohne müde zu machen und sind daher für den Einsatz tagsüber geeignet. Dies gilt insbesondere bei nervösen Unruhezuständen in der Prämenopause und/oder der Menopause.

Melissenextrakt wird pro Einheit in Mengen von 20 bis 120 mg eingesetzt. Denkbar sind auch Mengen von 30 bis 100 mg. Eine gängige Menge an Melissenextrakt beträgt 50 mg.

Yamswurzelextrakt enthält unter anderem folgende Inhaltsstoffe: Diosgenin, welches nach alternativer Medizin ein "natürliches" Progesteron darstellt. Damit ist Yamswurzelextrakt naturheilkundlich zur Linderung von Wechseljahresbeschwerden geeignet.

Yamswurzelextrakt wird pro Einheit in Mengen von 50 bis 300 mg eingesetzt. Denkbar sind auch Mengen von 100 bis 200 mg. Eine gängige Menge an Yamswurzelextrakt beträgt 150 mg.

Sojabohnenextrakt und Rotkleeextrakt enthalten unter anderem Isoflavone. Dazu gehören die Phytoöstrogene Genistein, Daidzei, Pratensein, Formononetin, Biochanin, die strukturell den Östrogenen ähneln und an die gleichen Rezeptoren binden. Vorteilhafterweise sind Nebenwirkungen, die unter Verabreichung von künstlichen Östrogenen auftreten, weniger ausgeprägt. Ohne an eine solche Theorie gebunden zu sein, können Isoflavone möglicherweise die Transkription des Enzyms COMT, das den Östrogenabbau fördert, hemmen. Durch Östrogenmangel bedingte Schlafstörungen, Nachtschweiß und nächtliche Hitzewallungen begründen die Einnahme am Abend.

Sojabohnenextrakt und Rotkleeextrakt werden vorzugsweise zusammen eingesetzt und dabei in einer Gesamtmenge, bezogen auf eine Einheit, von 50 bis 200 mg. Bevorzugt sind 80 bis 150 mg insgesamt und besonders bevorzugt 100 mg insgesamt. Gemäß einer besonderen Ausführungsform werden 50 mg Sojabohnenextrakt und 50 mg Rotkleeextrakt eingesetzt.

Extrakt aus der Eihaut eines Hühnereis (NEM® [Natural Egg Shell Membran]) enthält unter anderem Glucosamin, Chondroitinsulfat, Hyaluronsäure, Collagen Typ I, V und X, Elastin, Aminosäuren (wie Methionin und Cystein), Calcium, Proteine. Glucosamine und Chondroitinsulfat sind Bau- und Bestandteile von Bindegewebe, Knorpel, Gelenkflüssigkeit. In vitro konnten antiinflammatorische Wirkungen nachgewiesen werden. Hyaluronsäure ist Bestandteil von Knorpel und Gelenkflüssigkeit. Desweiteren enthält der Extrakt verschiedene Collagen-Typen (Strukturproteine des Bindegewebes bzw. der extrazellulären Matrix) sowie die Aminosäuren Methionin und Cystein, die zur Hemmung von proinflammatorischen Faktoren beitragen. Das enthaltene Calcium trägt zum Aufbau und Erhalt der Knochensubstanz bei. Die Einnahme von NEM® kann über einen längeren Zeitraum betrachtet die Gelenkbeweglichkeit verbessern, Schmerzen reduzieren und Entzündungen hemmen, was insbesondere tagsüber erwünscht ist.

NEM® wird in Mengen von 200 bis 800 mg, bezogen auf eine Einheit, eingesetzt. Denkbar sind Mengen von 300 bis 700 mg und eine gängige Menge an NEM® ist 500 mg.

### Die Vitamine

Die Einheiten der Verabreichungsform können Vitamine enthalten. Bevorzugte Vitamine sind ausgewählt aus der Gruppe bestehend aus Vitamin B₁, Vitamin B₂, Vitamin B₃, Vitamin B₅, Vitamin B₆, Vitamin B₇, Vitamin B₉, Vitamin B₁₂, Vitamin C, Vitamin D, Vitamin E, und Mischungen aus zweien oder mehreren daraus.

Die B-Vitamine können für eine verbesserte Durchblutung und somit für eine bessere Versorgung der Gewebe mit Sauerstoff sorgen. Dadurch können B-Vitamine zum Erhalt und zur Regeneration von Geweben beitragen. Die B-Vitamine sind aufgrund ihrer regenerativen Wirkung bevorzugt in Einheiten enthalten, die zur Einnahme am Abend vorgesehen sind.

Die B-Vitamine, insbesondere Vitamin B₁, Vitamin B₂, Vitamin B₃, Vitamin B₆ und Vitamin B₁₂, können ebenfalls einen regenerativen Einfluss auf das enterische Nervensystem ("Bauchhirn") haben. Das enterische Nervensystem ist ein eigenständiges Nervensystem, das als dünne Schicht zwischen den Muskeln des Verdauungsapparates ausgebildet ist. Das enterische Nervensystem umfasst vier- bis fünfmal mehr Neuronen als das Rückenmark. Das enterische Nervensystem ist autonom, wird aber beeinflusst vom Sympathikus und Parasympathikus. Das enterische Nervensystem kann einen starken Einfluss auf Verdauungsprozesse, insbesondere die Darmmotilität, lonentransporte bei Sekretion und Absorption sowie den gastrointestinalen Blutfluss und die immunologischen Funktionen des Gastrointestinaltraktes haben.

Vitamin E ist aufgrund seiner anti-oxidativen Wirkung bevorzugt in Einheiten enthalten, die zur Einnahme am Morgen vorgesehen sind. Bevorzugt enthalten Einheiten, die zur Einnahme am Morgen vorgesehen sind, mindestens eine Komponente mit anti-oxidativer Wirkung. Komponenten mit anti-oxidativer Wirkung sind bevorzugt ausgewählt aus der Gruppe bestehend aus Vitamin E, Mangan, Selen, Coenzym Q10 und Mischungen aus zweien oder mehreren daraus.

Coenzym Q10 ist als Bestandteil der Atmungskette auch an der Energiegewinnung beteiligt. Coenzym Q10 ist daher bevorzugt in Einheiten enthalten, die zur Einnahme mit dem Frühstück vorgesehen sind. In Einheiten, die zur Einnahme mit dem Abendessen vorgesehen sind, ist Coenzym Q10 bevorzugt nicht enthalten.

Vitamin B₁ (Thiamin) ist als Thiamindiphosphat Co-Enzym an zentraler Stelle im Energie-/Kohlehydratstoffwechsel beteiligt. Vitamin B₁ trägt zu einer normalen Herzfunktion bei. Vitamin B₁ trägt zu einer normalen Funktion des Nervensystems bei. Vitamin B₁ trägt zur normalen psychischen Funktion bei. Vitamin B₁ ist an Energiegewinnung (insbes. Herzmuskel, Gehirn), an Reizweiterleitung im Nervensystem, am Metabolismus wichtiger Neurotransmitter (z. B. Acetylcholin, Serotonin) und an Blutbildung beteiligt. Vitamin B₁ ist bevorzugt in Einheiten enthalten, die zur Einnahme mit dem Abendessen vorgesehen sind. In Einheiten, die zur Einnahme mit dem Frühstück vorgesehen sind, ist Vitamin B₁ bevorzugt nicht enthalten.

Vitamin B₂ (Riboflavin) ist an verschiedenen Stoffwechselprozessen, hormonellen Regulationen und der Hämatopoese beteiligt. Vitamin B₂ trägt zu einer normalen Funktion des Nervensystems bei. Vitamin B₂ ist an der Energiegewinnung (Atmungskette) in jeder Körperzelle, am Auf- und Abbau roter Blutkörperchen und am Erhalt der Myelinschicht (Ummantelung von Nervenfasern) beteiligt. Vitamin B₂ ist bevorzugt in Einheiten enthalten, die zur Einnahme mit dem Abendessen vorgesehen sind. In Einheiten, die zur Einnahme mit dem Frühstück vorgesehen sind, ist Vitamin B₂ bevorzugt nicht enthalten.

Vitamin B₃ (Niacin) ist an verschiedenen Stoffwechselprozessen, hormonellen Regulationen und der Hämatopoese beteiligt. Vitamin B₃ trägt zu einer normalen Funktion des Nervensystems bei. Vitamin B₃ trägt zur normalen psychischen Funktion bei. Vitamin B₃ ist an der Energiegewinnung und am Aufbau von Fettsäuren und Cholesterol beteiligt. Vitamin B₃ ist bevorzugt in Einheiten enthalten, die zur Einnahme mit dem Abendessen vorgesehen sind. In Einheiten, die zur Einnahme mit dem Frühstück vorgesehen sind, ist Vitamin B₃ bevorzugt nicht enthalten.

Vitamin B₅ (Pantothensäure) ist an verschiedenen Stoffwechselprozessen, hormonellen Regulationen und der Hämatopoese beteiligt. Vitamin B₅ trägt zu einer normalen Synthese und zu einem normalen Stoffwechsel von Steroidhormonen, Vitamin D und einigen Neurotransmittern bei. Vitamin B₅ trägt zu einer normalen geistigen Leistung bei. Vitamin B₅ ist am Auf- und Abbau von Kohlenhydraten und an der Steroidsynthese beteiligt. Vitamin B₅ ist bevorzugt in Einheiten enthalten, die zur Einnahme mit dem Abendessen vorgesehen sind. In Einheiten, die zur Einnahme mit dem Frühstück vorgesehen sind, ist Vitamin B₅ bevorzugt nicht enthalten.

Vitamin B₆ ist als Co-Enzym bei ca. 100 enzymatischen Reaktionen im Aminosäure-Stoffwechsel beteiligt. Vitamin B₆ trägt zu einer normalen Cystein-Synthese bei. Vitamin B₆ trägt zu einem normalen Homocystein-Stoffwechsel bei. Vitamin B₆ ist auch Co-Faktor bei der Häm-Synthese. Vitamin B₆ trägt also zur normalen Bildung roter Blutkörperchen bei. Vitamin B₆ trägt zur Regulierung der Hormontätigkeit bei. Vitamin B₆ trägt zur normalen psychischen Funktion bei. Vitamin B₆ trägt zu einer normalen Funktion des Nervensystems bei. Vitamin B₆ ist auch am Neurotransmitterstoffwechsel beteiligt. Vitamin B₆ trägt auch zu einem normalen Glycogenstoffwechsel bei. Vitamin B₆ ist bevorzugt in Einheiten enthalten, die zur Einnahme mit dem Abendessen vorgesehen sind. In Einheiten, die zur Einnahme mit dem Frühstück vorgesehen sind, ist Vitamin B₆ bevorzugt nicht enthalten.

Vitamin B₇ (Biotin) ist an der Neusynthese von D-Glucose beteiligt. Dadurch trägt Vitamin B₇ zu einer normalen Funktion des Nervensystems bei. Vitamin B₇ trägt also auch zur normalen psychischen Funktion bei. Vitamin B₇ ist bevorzugt in Einheiten enthalten, die zur Einnahme mit dem Abendessen vorgesehen sind. In Einheiten, die zur Einnahme mit dem Frühstück vorgesehen sind, ist Vitamin B₇ bevorzugt nicht enthalten.

Vitamin B₉ (Folsäure) ist als Vorstufe des Coenzyms Tetrahydrofolsäure an der Umwandlung von Homocystein zu Methionin beteiligt. Da ein erhöhter Homocystein-Spiegel zu einer toxischen Schädigung von Gefäßwänden und somit zu einem erhöhten Risiko von Herz-Kreislauf-Erkrankungen, insbesondere zu einer erhöhten Thrombose-Neigung, führen kann, trägt Vitamin B₉ zu einem gesunden Herz-KreislaufSystem bei. Ein Mangel an Vitamin B₉ wirkt sich auf das Blutbild aus (hyperchrome makrozytäre Anämie). Symptome sind unter anderem Schwäche, Konzentrationsschwäche, Irritationen, Kopfschmerzen, Palpitationen und Kurzatmigkeit. Ein erhöhter Homocystein-Spiegel kann auch zu Demenz und der Alzheimer-Erkrankung beitragen. Vitamin B₉ trägt also zur normalen psychischen Funktion bei. Vitamin B₉ ist bevorzugt in Einheiten enthalten, die zur Einnahme mit dem Abendessen vorgesehen sind. In Einheiten, die zur Einnahme mit dem Frühstück vorgesehen sind, ist Vitamin B₉ bevorzugt nicht enthalten. In alternativen Ausführungsformen kann Vitamin B₉ jedoch auch in Einheiten enthalten sein, die zur Einnahme mit dem Frühstück vorgesehen sind.

Auch Vitamin B₁₂ trägt zu einem normalen Homocystein-Stoffwechsel bei. Außerdem ist Vitamin B₁₂ Bestandteil des Fettsäure-, Kohlenhydrat- und Nukleinsäure-Stoffwechsels und ist beteiligt an der Myelinsynthese. Vitamin B₁₂ ist auch beteiligt an der Hämatopoese. Vitamin B₁₂ trägt zu einer normalen Bildung roter Blutkörperchen bei. Vitamin B₁₂ trägt zu einer normalen Funktion des Nervensystems bei. Vitamin B₁₂ trägt zur normalen psychischen Funktion bei. Vitamin B₁₂ trägt zu einer normalen Funktion des Nervensystems bei. Vitamin B₁₂ ist bevorzugt in Einheiten enthalten, die zur Einnahme mit dem Abendessen vorgesehen sind. In Einheiten, die zur Einnahme mit dem Frühstück vorgesehen sind, ist Vitamin B₁₂ bevorzugt nicht enthalten.

Vitamin C trägt zu einer normalen Kollagenbildung bei. Dadurch trägt Vitamin C zu einer normalen Knochen- und Knorpelfunktion bei. Aufgrund seiner regenerativen und aufbauenden Wirkung kann Vitamin C in Einheiten enthalten sein, die zur Einnahme am Abend vorgesehen sind. Aufgrund seiner antioxidativen Wirkungen kann Vitamin C auch in Einheiten, die zur Einnahme mit dem Frühstück vorgesehen sind, verwendet werden.

Vitamin D trägt zu einem normalen Calciumspiegel im Blut und dadurch zur Erhaltung normaler Knochen bei. Eine wichtige Funktion von Vitamin D ist also die Calcium- (und Phosphat-) Homöostase. Vitamin D fördert die Calcium-Aufnahme im Darm und hemmt die Calcium-Ausscheidung durch die Nieren. Durch die Erhöhung des Blutcalcium-Spiegels wird ein vermehrter Aufbau neuer Knochensubstanz durch Osteoblasten erreicht. Aufgrund seiner regenerativen und aufbauenden Wirkung ist Vitamin D bevorzugt in Einheiten enthalten, die zur Einnahme am Abend vorgesehen sind. In Einheiten, die zur Einnahme mit dem Frühstück vorgesehen sind, ist Vitamin D bevorzugt nicht enthalten. Da Vitamin D die Calcium-Aufnahme im Darm fördert, kann Vitamin D jedoch in alternativen Ausführungsformen auch in Einheiten enthalten sein, die zur Einnahme mit dem Frühstück vorgesehen sind.

Vitamin E trägt dazu bei, Zellen vor oxidativem Stress zu schützen. Vitamin E ist bevorzugt in Einheiten enthalten, die zur Einnahme mit dem Frühstück vorgesehen sind. In Einheiten, die zur Einnahme mit dem Abendessen vorgesehen sind, ist Vitamin E bevorzugt nicht enthalten.

### Die Mineralstoffe

Die Einheiten der Verabreichungsform können Mineralstoffe enthalten. Bevorzugte Mineralstoffe sind ausgewählt aus der Gruppe bestehend aus Zink, Eisen, Magnesium, Calcium, Mangan, Selen, Silizium und Mischungen aus zweien oder mehreren daraus.

Mangan ist ein essentielles Spurenelement. Mangan ist ein Co-Faktor für Enzyme des Aufbaus von Knorpel- und Knochengewebe. Dadurch trägt Mangan zur Erhaltung normaler Knochen bei. Mangan ist ein Co-Faktor für das Enzym Superoxiddismutase, das der "Neutralisierung" reaktiver Sauerstoffspezies dient. Dadurch trägt Mangan dazu bei, Zellen vor oxidativem Stress zu schützen. Mangan ist daher bevorzugt in Einheiten enthalten, die zur Einnahme mit dem Frühstück vorgesehen sind. In Einheiten, die zur Einnahme mit dem Abendessen vorgesehen sind, ist Mangan bevorzugt nicht enthalten.

Selen ist ein essentielles Spurenelement. Selen ist ein Radikalfänger. Dadurch trägt Selen dazu bei, Zellen vor oxidativem Stress zu schützen. Selen ist daher bevorzugt in Einheiten enthalten, die zur Einnahme mit dem Frühstück vorgesehen sind. In Einheiten, die zur Einnahme mit dem Abendessen vorgesehen sind, ist Selen bevorzugt nicht enthalten.

Calcium dient der Mineralisierung von Knochen und Zähnen als Hydroxylapatit (Ca₅(PO₄)₃(OH)). Calcium wird für die Erhaltung normaler Knochen benötigt. Aufgrund seiner regenerativen und aufbauenden Wirkung ist Calcium bevorzugt in Einheiten enthalten, die zur Einnahme am Abend vorgesehen sind. Allerdings kann Calcium in alternativen Ausführungsformen auch in Einheiten enthalten sein, die zur Einnahme mit dem Frühstück vorgesehen sind. Vitamin D, das unter anderem unter Lichteinfluss gebildet wird, fördert die Calcium-Aufnahme im Darm. Verschiedene Verdauungsenzyme (z.B. Lipasen) benötigen Calcium zur Aktivierung. Calcium trägt also neben der Erhaltung der Knochen zur normalen Funktion von Verdauungsenzymen bei. Gemäß einer bevorzugten Ausführungsform der vorliegenden Erfindung enthalten daher Einheiten, die Calcium enthalten, zusätzlich auch Vitamin D.

Zink ist ein Co-Faktor für Enzyme des Knochenaufbaus. Zink stimuliert Knochenbildung und Mineralisation durch Osteoblasten. Zink inhibiert Knochenabbau und Demineralisation durch Osteoclasten. Dadurch trägt Zink zur Erhaltung normaler Knochen bei. Zink wird gespeichert in Vesikeln glutaminerger Synapsen. Zink kann Rezeptoraktivitäten im zentralen Nervensystem modulieren. Zink-Supplementierung kann kognitive Funktionen verbessern. Zink trägt zu einer normalen kognitiven Funktion bei. Zink ist bevorzugt in Einheiten enthalten, die zur Einnahme mit dem Frühstück vorgesehen sind. In Einheiten, die zur Einnahme mit dem Abendessen vorgesehen sind, ist Zink bevorzugt nicht enthalten.

Magnesium ist ein Co-Faktor von Enzymen. Magnesium trägt zu einer normalen Muskelfunktion bei. Magnesium-Mangel kann zu allgemeinem Schwächegefühl, Herzrhythmusstörungen und Muskelkrämpfen führen, wobei besonders häufig nächtliche Wadenkrämpfe beobachtet werden. Magnesium ist daher bevorzugt in Einheiten enthalten, die zur Einnahme mit dem Abendessen vorgesehen sind. In Einheiten, die zur Einnahme mit dem Frühstück vorgesehen sind, ist Magnesium bevorzugt nicht enthalten.

Eisen dient dem Transport und der Speicherung von Sauerstoff (Bestandteil Hämoglobin) und wird auch in der Atmungskette (Energiegewinnung) benötigt. Durch seinen Beitrag zur Sauerstoffversorgung trägt Eisen zu einer normalen kognitiven Funktion bei. Eisen ist bevorzugt in Einheiten enthalten, die zur Einnahme mit dem Abendessen vorgesehen sind. In Einheiten, die zur Einnahme mit dem Frühstück vorgesehen sind, ist Eisen bevorzugt nicht enthalten.

Bezüglich der Mengen der eingesetzten Substanzen wird der Fachmann geeignete Mengen gemäß Lebensmittelinformationsverordnung, geltend zum Anmeldetag dieser Anmeldung wählen, wobei mindestens 16% und höchstens 300% der empfohlenen Tagesdosis gewählt werden.

Vorzugsweise beträgt das Massenverhältnis von eingesetzten Pilzkomponenten zu den eingesetzten Pflanzenextrakten (gemeint ist jeweils die Summe aller in einer Einheit einer Verabreichungsform eingesetzten Pilzkomponenten und Pflanzenextrakte) mindestens 1:2, bevorzugt mindestens 1:1, weiter bevorzugt mindestens 2:1 und weiter bevorzugt 5:1.

Vorzugsweise beträgt das Massenverhältnis von eingesetzten Pilzkomponenten zu den eingesetzten Vitaminen (gemeint ist jeweils die Summe aller in einer Einheit einer Verabreichungsform eingesetzten Pilzkomponenten und Vitamine) mindestens 2:1, bevorzugt mindestens 5:1, weiter bevorzugt mindestens 10:1 und weiter bevorzugt 100:1.

Vorzugsweise beträgt das Massenverhältnis von eingesetzten Pilzkomponenten zu den eingesetzten Mineralstoffen (gemeint ist jeweils die Summe aller in einer Einheit einer Verabreichungsform eingesetzten Pilzkomponenten und Mineralstoffe) mindestens 1:1, bevorzugt mindestens 10:1, weiter bevorzugt mindestens 20:1 und weiter bevorzugt 50:1.

### Hilfsstoffe

Der Fachmann wird pharmazeutisch übliche Hilfsstoffe, wie insbesondere mikrokristalline Cellulose, in den Einheiten der Verabreichungsformen einsetzen. Je nach Ausgestaltung der Verabreichungsform wählt der Fachmann gemäß seinem Fachwissen nötige Hilfsstoffe, wie beispielsweise auch Überzüge für Dragees.

### Zu den Beispielen

Der Fachmann wird den Beispielen entnehmen, dass sich die unterschiedliche Zusammensetzung der Einheiten A und B der Verabreichungsform auch aufgrund unterschiedlicher Pflanzenextrakte, und/oder aufgrund unterschiedlicher Vitamine und/oder aufgrund unterschiedlicher Mineralstoffe ergeben kann. Wie weiter oben zur Pilzkomponente ausgeführt kann der Unterschied jeweils auch nur durch eine unterschiedliche Menge der jeweiligen Substanz bedingt sein.

### Beispiele

### Beispiel A -Kapseln zur Steigerung der Gedächtnisleistung

Zusammensetzung 1 zur Einnahme am Morgen (kann als Einheit A der Verabreichungsform bezeichnet werden)

| **Rohstoffe** | **ausgelobter Stoff** | **ausgelobter Stoff in mg / Kps.** | **mg / Kps.** | **g / 100 g Füllgewicht** |
|---|---|---|---|---|
| Cordyceps sinensis CS-4 Extrakt | | 200 | 200,00 | 35,6888 |
| Ginseng Extrakt | | 100 | 100,00 | 17,8444 |
| Rhodiola rosea Extrakt | | 150 | 150,00 | 26,7666 |
| Folsäure | Folsäure | 0,4 | 0,40 | 0,0714 |
| Zinkgluconat | Zink | 10 | 70,00 | 12,4911 |
| Mikrokristalline Cellulose Ph. Eur. (Vivapur 102) | | | 40,00 | 7,1378 |
| | | Kapselfüllgewicht | 560,40 | 100,0000 |
| | | Kapselleergewicht | 122,00 | |
| | | Kapselsollgewicht | 682,40 | |

Zusammensetzung 2 zur Einnahme am Abend (kann als Einheit B der Verabreichungsform bezeichnet werden)

| **Rohstoffe** | **ausgelobter Stoff** | **ausgelobter Stoff in mg / Kps.** | **mg / Kps.** | **g / 100 g Füllgewicht** |
|---|---|---|---|---|
| Hericium erinaceus Extrakt | | 300 | 300,00 | 60,5498 |
| Ginkgo Extrakt | | 80 | 80,00 | 16,1466 |
| Vitamin B1 HCl | Thiamin | 2,2 | 3,00 | 0,6055 |
| Vitamin B2 | Riboflavin | 2,8 | 2,80 | 0,5651 |
| Vitamin B6 HCl | Vitamin B6 | 2,8 | 3,46 | 0,6983 |
| Vitamin B12, Verreibung 1 % | Vitamin B 12 | 0,005 | 0,50 | 0,1009 |
| Nicotinamid | Niacin | 32 | 32,00 | 6,4586 |
| Calcium-d-Pantothenat | Panthothensäure | 12 | 13,10 | 2,6440 |
| Biotin | Biotin | 0,1 | 0,10 | 0,0202 |
| Eisen(II)gluconat x H2O | Eisen | 7 | 60,50 | 12,2109 |
| Mikrokristalline Cellulose Ph. Eur. (Vivapur 102) | | | 0,00 | 0,0000 |
| | | Kapselfüllgewicht | 495,46 | 100,0000 |
| | | Kapselleergewicht | 122,00 | |
| | | Kapselsollgewicht | 617,46 | |

### Beispiel B -Kapseln zur Stärkung des Herz-Kreislauf-Systems

Zusammensetzung 3 zur Einnahme am Morgen (kann als Einheit A der Verabreichungsform bezeichnet werden)

| **Rohstoffe** | **ausgelobter Stoff** | **ausgelobter Stoff in mg / Kps** | **mg / Kps** | **g / 100 g Füllgewicht** |
|---|---|---|---|---|
| Maitake Extrakt | | 230 | 230,00 | 42,1245 |
| Weintraubenkernextrakt | | 200 | 200,00 | 36,6300 |
| Coenzym Q10 | Coenzym Q10 | 30 | 30,00 | 5,4945 |
| Vitamin E 700, Zubereitung 50 % | Vitamin E | 18 | 36,00 | 6,5934 |
| Mikrokristalline Cellulose Ph. Eur. (Vivapur 102) | | | 50,00 | 9,1575 |
| | | Kapselfüllgewicht | 546,00 | 100,0000 |
| | | Kapselleergewicht | 121,99 | |
| | | Kapselsollgewicht | 667,99 | |

Zusammensetzung 4 zur Einnahme am Abend (kann als Einheit B der Verabreichungsform bezeichnet werden)

| **Rohstoffe** | **ausgelobter Stoff** | **ausgelobter Stoff in mg / Kps.** | **mg / Kps.** | **g / 100g Füllgewicht** |
|---|---|---|---|---|
| Auricularia Extrakt | | 210 | 210,00 | 42,1619 |
| Weißdornextrakt | | 100 | 100,00 | 20,0771 |
| Magnesiumoxid schwer | Magnesium | 80 | 132,67 | 26,6363 |
| Vitamin B6 HCl | Vitamin B6 | 2,8 | 3,46 | 0,6947 |
| Vitamin B1 HCl | Thiamin | 1,1 | 1,50 | 0,3012 |
| Vitamin B12, Verreibung 1 % | Vitamin B12 | 0,0025 | 0,25 | 0,0502 |
| Folsäure | Folsäure | 0,2 | 0,20 | 0,0402 |
| Mikrokristalline Cellulose Ph. Eur. (Vivapur 102) | | | 50,00 | 10,0385 |
| | | Kapselfüllgewicht | 498,08 | 100,0000 |
| | | Kapselleergewicht | 118,00 | |
| | | Kapselsollgewicht | 616,08 | |

### Beispiel C -Kapseln für einen gesunden Maaen-Darm-Trakt

Zusammensetzung 5 zur Einnahme am Morgen (kann als Einheit A der Verabreichungsform bezeichnet werden)

| **Rohstoffe** | **ausgelobter Stoff** | **ausgelobter Stoff in mg / Kps.** | **mg / Kps.** | **g / 100 g Füllgewicht** |
|---|---|---|---|---|
| Hericium erinaceus Pulver | | 120 | 120,00 | 16,7364 |
| Artischockenextrakt, DEV 12:1 | | 270 | 270,00 | 37,6569 |
| Vitamin D3, Zubereitung 100 000 IU/g | Vitamin D | 0,005 | 2,00 | 0,2789 |
| Calciumcarbonat schwer | Calcium | 130 | 325,00 | 45,3278 |
| Mikrokristalline Cellulose Ph. Eur. (Vivapur 102) | | | 0,00 | 0,0000 |
| | | Kapselfüllgewicht | 717,00 | 100,0000 |
| | | Kapselleergewicht | 118,00 | |
| | | Kapselsollgewicht | 835,00 | |

Zusammensetzung 6 zur Einnahme am Abend (kann als Einheit B der Verabreichungsform bezeichnet werden)

| **Rohstoffe** | **ausgelobter Stoff** | **ausgelobter Stoff in mg / Kps.** | **mg / Kps.** | **g / 100 g Fülgewicht** |
|---|---|---|---|---|
| Hericium erinaceus Extrakt | | 270 | 270,00 | 54,7312 |
| Fenchelextrakt DEV 4:1 | | 200 | 200,00 | 40,5416 |
| Vitamin B1 HCl | Thiamin | 1,65 | 2,25 | 0,4561 |
| Vitamin B2 | Riboflavin | 2,1 | 2,10 | 0,4257 |
| Nicotinamid | Niacin | 16 | 16,00 | 3,2433 |
| Vitamin B6 HCl | Vitamin B6 | 2,1 | 2,60 | 0,5270 |
| Vitamin B12, Zubereitung 1 % | Vitamin B12 | 0,0037 | 0,37 | 0,0750 |
| Mikrokristalline Cellulose Ph. Eur. (Vivapur 102) | | | 0,00 | 0,0000 |
| | | Kapselfüllgewicht | 493,32 | 100,0000 |
| | | Kapselleergewicht | 118,01 | |
| | | Kapselsollgewicht | 611,33 | |

### Beispiel D -Kapseln für hormonelle Balance in den Wechseljahren

Zusammensetzung 7 zur Einnahme am Morgen (kann als Einheit A der Verabreichungsform bezeichnet werden)

| **Rohstoffe** | **ausgelobter Stoff** | **ausgelobter Stoff in mg / Kps.** | **mg / Kps.** | **g / 100 g Füllgewicht** |
|---|---|---|---|---|
| Cordyceps sinensis CS-4 Extrakt | | 200 | 200,00 | 34,7826 |
| Melissenextrakt, DEV 10:1 | | 50 | 50,00 | 8,6957 |
| Yamswurzelextrakt | | 150 | 150,00 | 26,0870 |
| Coenzym Q10 | Coenzym, Q10 | 25 | 25,00 | 4,3478 |
| Mikrokristalline Cellulose Ph. Eur. (Vivapur 102) | | | 150,00 | 26,0870 |
| | | Kapselfüllgewicht | 575,00 | 100,0000 |
| | | Kapselleergewicht | 122,00 | |
| | | Kapselsollgewicht | 697,00 | |

Zusammensetzung 8 zur Einnahme am Abend (kann als Einheit B der Verabreichungsform bezeichnet werden)

| **Rohstoffe** | **ausgelobter Stoff** | **ausgelobter Stoff in mg / Kps.** | **mg / Kps.** | **g / 100g Füllgewicht** |
|---|---|---|---|---|
| Maitake Extrakt | | 200 | 200,00 | 37,9557 |
| Sojaextrakt | | 50 | 50,00 | 9,4889 |
| Rotkleeextrakt | | 50 | 50,00 | 9,4889 |
| Vitamin B6 HCl | Vitamin B6 | 1,4 | 1,73 | 0,3283 |
| Nicotinamid | Niacin | 16 | 16,00 | 3,0365 |
| Calcium-d-Pantothenat | Pantothensäure | 6 | 6,55 | 1,2430 |
| Vitamin B2 | Riboflavin | 1,4 | 1,40 | 0,2657 |
| Vitamin B12 (Zubereitung 1 %) | Vitamin B12 | 0,0025 | 0,25 | 0,0474 |
| Eisen(II)gluconat x H2O | Eisen | 14 | 121,00 | 22,9632 |
| Mikrokristalline Cellulose Ph. Eur. (Vivapur 102) | | | 80,00 | 15,1823 |
| | | Kapselfüllgewicht | 526,93 | 100,00 |
| | | Kapselleergewicht | 122,01 | |
| | | Kapselsollgewicht | 648,94 | |

### Beispiel E -Kapseln für Gelenke und Knochen

Zusammensetzung 9 zur Einnahme am Morgen (kann als Einheit A der Verabreichungsform bezeichnet werden)

| **Rohstoffe** | **ausgelobter Stoff** | **ausgelobter Stoff in mg / Kps.** | **mg / Kps.** | **g / 100 g Füllgewicht** |
|---|---|---|---|---|
| Maitake Extrakt | | 140 | 140,00 | 26,5222 |
| NEM^{®} Extrakt | | 250 | 250,00 | 47,3610 |
| Vitamin E 700, Zubereitung 50 % | Vitamin E | 6 | 12,00 | 2,2733 |
| Mangan(II)gluconat | Mangan | 1 | 8,11 | 1,5364 |
| Natriumselenat, Zubereitung 1 % Se | Selen | 0,0275 | 2,75 | 0,5210 |
| Zinkgluconat | Zink | 5 | 35,00 | 6,6305 |
| Mikrokristalline Cellulose Ph. Eur. (Vivapur 102) | | | 80,00 | 15,1555 |
| | | Kapselfüllgewicht | 527,86 | 100,0000 |
| | | Kapselleergewicht | 122,00 | |
| | | Kapselsollgewicht | 649,86 | |

Zusammensetzung 10 zur Einnahme am Abend (kann als Einheit B der Verabreichungsform bezeichnet werden)

| **Rohstofffe** | **ausgelobter Stoff** | **ausgelobter Stoff in mg / Kps.** | **mg / Kps.** | **g / 100 g Füllgewicht** |
|---|---|---|---|---|
| Shiitake Extrakt | | 140 | 140,00 | 24,3056 |
| Hagebuttenextrakt, 40% Vitamin C | | 210 | 210,00 | 36,4583 |
| Calciumcarbonat schwer | Calcium | 30 | 75,00 | 13,0208 |
| Vitamin D3, Zubereitung 100 000 IU/g | Vitamin D | 0,0025 | 1,00 | 0,1736 |
| Mikrokristalline Cellulose Ph. Eur. (Vivapur 102) | | | 150,00 | 26,0417 |
| | | Kapselfüllgewicht | 576,00 | 100,0000 |
| | | Kapselleergewicht | 122,00 | |
| | | Kapselsollgewicht | 698,00 | |

### Beispiel F -Kapselzusammensetzungen der Beispiele A bis E

Zusammensetzung der Kapseln der Zusammensetzung 1

| **Kapseln** | mg / Kps. | g / 100 g Gesamtgew. | |
|---|---|---|---|
| HPMC, E464 | 121,0342 | 17,74 | Kapselhülle |
| Titandioxid, E171 | 0,9550 | 0,14 | Farbstoff |
| Brilliant blue FCF - FD&C Blue 1, E 133 | 0,0108 | 0,00 | Farbstoff |
| Kapselleergewicht | 122,00 | 17,88 | |

Zusammensetzung der Kapseln der Zusammensetzung 2

| **Kapseln** | mg / Kps. | g / 100 g Gesamtgew. | |
|---|---|---|---|
| HPMC, E464 | 120,9362 | 19,41 | Kapselhülle |
| Titandioxid, E171 | 0,9760 | 0,16 | Farbstoff |
| Brilliant blue FCF - FD&C Blue 1, E 133 | 0,087 | 0,01 | Farbstoff |
| Kapselleergewicht | 122,00 | 19,58 | |

Zusammensetzung der Kapseln der Zusammensetzung 3

| **Kapseln** | mg / Kps. | g / 100 g Gesamtgew. | |
|---|---|---|---|
| HPMC, E464 | 121,1 | 18,0318 | Kapselhülle |
| Titandioxid, E171 | 0,6 | 0,0893 | Farbstoff |
| Red Iron Oxide, E172 | 0,26 | 0,0387 | Farbstoff |
| Yellow Iron Oxide, E172 | 0,03 | 0,0045 | Farbstoff |
| Kapselleergewicht | 121,99 | 18,1644 | |

Zusammensetzung der Kapseln der Zusammensetzung 4

| **Kapseln** | mg / Kps. | g / 100 g Gesamtgew. | |
|---|---|---|---|
| HPMC, E464 | 117,29 | 19,0214 | Kapselhülle |
| Red Iron Oxide, E172 | 0,25 | 0,0405 | Farbstoff |
| Black Iron Oxide, E 172 | 0,21 | 0,0341 | Farbstoff |
| Yellow Iron Oxide, E172 | 0,18 | 0,0292 | Farbstoff |
| Titandioxid, E171 | 0,07 | 0,0114 | Farbstoff |
| Kapselleergewicht | 118,00 | 19,1366 | |

Zusammensetzung der Kapseln der Zusammensetzung 5

| **Kapseln** | mg / Kps. | g / 100 g Gesamtgew. | |
|---|---|---|---|
| HPMC, E464 | 117,25 | 14,0386 | Kapselhülle |
| Titandioxid, E171 | 0,71 | 0,0850 | Farbstoff |
| Copper complex of chlorophyllins, E141 | 0,04 | 0,0048 | Farbstoff |
| Kapselleergewicht | 118,00 | 14,1284 | |

Zusammensetzung der Kapseln der Zusammensetzung 6

| **Kapseln** | mg / Kps. | g / 100 g Gesamtgew. | |
|---|---|---|---|
| HPMC, E464 | 116,87 | 19,0447 | Kapselhülle |
| Titandioxid, E171 | 0,91 | 0,1483 | Farbstoff |
| Copper complex of chlorophyllins, E141 | 0,23 | 0,0375 | Farbstoff |
| Kapselleergewicht | 118,01 | 19,2305 | |

Zusammensetzung der Kapseln der Zusammensetzung 7

| **Kapseln** | mg / Kps. | g / 100 g Gesamtgew. | |
|---|---|---|---|
| HPMC, E464 | 121,25 | 17,3960 | Kapselhülle |
| Titandioxid, E171 | 0,73 | 0,1047 | Farbstoff |
| Red Iron Oxide, E 172 | 0,02 | 0,0029 | Farbstoff |
| Kapselleergewicht | 122,00 | 17,5036 | |

Zusammensetzung der Kapseln der Zusammensetzung 8

| **Kapseln** | mg / Kps. | g / 100 g Gesamtgew. | |
|---|---|---|---|
| HPMC, E464 | 120,97 | 18,5670 | Kapselhülle |
| Candurin Silver Fine E171 (E555) | 0,98 | 0,1504 | Farbstoff |
| Allura red AC-FD&C Red 40 E 129 | 0,04 | 0,0061 | Farbstoff |
| Brilliant blue FCF-FD&C Blue E133 | 0,02 | 0,0031 | Farbstoff |
| Kapselleergewicht | 122,01 | 18,7266 | |

Zusammensetzung der Kapseln der Zusammensetzung 9

| **Kapseln** | mg / Kps. | g / 100 g Gesamtgew. | |
|---|---|---|---|
| HPMC, E464 | 120,8507 | 18,5621 | Kapselhülle |
| Titandioxid, E171 | 1,0931 | 0,1679 | Farbstoff |
| Yellow Iron Oxide, E172 | 0,0562 | 0,0086 | Farbstoff |
| Kapselleergewicht | 122,00 | 18,7387 | |

Zusammensetzung der Kapseln der Zusammensetzung 10

| **Kapseln** | mg / Kps. | g / 100 g Gesamtgew. | |
|---|---|---|---|
| HPMC, E464 | 121,02 | 17,3356 | Kapselhülle |
| Titandioxid, E171 | 0,49 | 0,0702 | Farbstoff |
| Yellow Iron Oxide, E172 | 0,49 | 0,0702 | Farbstoff |
| Kapselleergewicht | 122,00 | 17,4760 | |

## Patentansprüche

1. Verabreichungsform umfassend zwei Einheiten A und B, die unabhängig voneinander verabreicht werden können, wobei die Einheiten A und B jeweils eine Pilzkomponente enthalten und ferner mindestens zwei weitere Substanzen ausgewählt aus Naturstoffextrakten, Vitaminen und Mineralstoffen, wobei sich die Einheiten A und B bezüglich ihrer Zusammensetzung voneinander unterscheiden.

2. Verabreichungsform gemäß Anspruch 1, wobei es sich bei den Pilzkomponenten um Pilzextrakte aus chinesischen Pilzen oder um Pilzpulver chinesischer Pilze handelt.

3. Verabreichungsform gemäß Anspruch 1 oder 2, wobei die Pilzkomponente ein Pulver oder ein Extrakt eines Pilzes ist und ausgewählt ist aus der Gruppe bestehend aus Cordyceps sinensis, Shiitake, Maitake, Auricularia polytricha, Hericium erinaceus, Coprinus comatus, Coriolus versicolor, Pleurotus ostreatus, Polyporus umbellatus, Reishi-Ganoderma lucidum, Agaricus blazei Murrill und Mischungen aus zwei oder mehreren daraus.

4. Verabreichungsform gemäß einem der vorhergehenden Ansprüche, wobei die Naturstoffextrakte Pflanzenextrakte oder Extrakte aus tierischen Produkten sind.

5. Verabreichungsform gemäß Anspruch 4, wobei die Pflanzenextrakte ausgewählt sind aus der Gruppe bestehend aus Hagebuttenextrakt, Traubenkernextrakt, Weißdornfruchtextrakt, Melissenextrakt, Yamswurzelextrakt, Sojaextrakt, Rotkleeextrakt, Ginsengextrakt, Rosenwurzextrakt, Artischockenextrakt, Fenchelextrakt und Ginkgoextrakt, Bambussprossenextrakt, Haferextrakt, Brennesselextrakt, Brokkoliextrakt und Mischungen aus zwei oder mehreren daraus.

6. Verabreichungsform gemäß einem der vorhergehenden Ansprüche, wobei die Vitamine ausgewählt sind aus der Gruppe bestehend aus Vitamin B₁, Vitamin B₂, Vitamin B₃, Vitamin B₅, Vitamin B₆, Vitamin B₇, Vitamin Bg, Vitamin B₁₂, Vitamin C, Vitamin D, Vitamin E, und Mischungen aus zweien oder mehreren daraus.

7. Verabreichungsform gemäß einem der vorhergehenden Ansprüche, wobei die Mineralstoffe ausgewählt sind aus der Gruppe bestehend aus Zink, Eisen, Magnesium, Calcium, Mangan, Selen, Silizium und Mischungen aus zweien oder mehreren daraus.

8. Verabreichungsform gemäß einem der vorhergehenden Ansprüche, wobei die Menge an Pilzkomponente 50 mg bis 400 mg, bezogen auf eine Einheit, beträgt.

9. Verabreichungsform gemäß einem der vorhergehenden Ansprüche, wobei eine oder beide Einheit/en der Verabreichungsform fest ist/sind.

10. Verabreichungsform gemäß einem der vorhergehenden Ansprüche, wobei mindestens eine Einheit oder beide Einheiten der Verabreichungsform oral verabreicht wird/werden.

11. Verabreichungsform gemäß einem der vorhergehenden Ansprüche, wobei mindestens eine Einheit oder beide Einheiten der Verabreichungsform eine Kapsel ist/sind.

12. Verwendung einer Verabreichungsform nach einem der Ansprüche 1 bis 11 zur Steigerung des Wohlbefindens eines Menschen, wobei die Einheiten A und B zu unterschiedlichen Tageszeiten eingenommen werden.

13. Verwendung gemäß Anspruch 12, wobei zwischen der oralen Einnahme der Einheit A und der oralen Einnahme der Einheit B zwischen 6 und 15 Stunden an einem Tag liegen.
